# EUROPÄISCHE PATENTSCHRIFT

(11) **EP 0 736 518 B1**
(45) Veröffentlichungstag und Bekanntmachung des Hinweises auf die Patenterteilung: **02.06.1999**
(21) Anmeldenummer: 96104870.9
(22) Anmeldetag: 27.03.1996
(51) Int. Cl.: C07C 209/86

(54) **Fraktionierung und Reinigung von aromatischen Polyamingemischen und deren Verwendung**
Fractionation and purification of mixtures of aromatic polyamines and their use
Fractionnement et purification de mélanges de polyamines aromatiques et leur utilisation

(30) Priorität: 07.04.1995 DE 19513068
(43) Veröffentlichungstag der Anmeldung: 09.10.1996
(73) Patentinhaber: BAYER AG, 51368 Leverkusen (DE)
(72) Erfinder: Knöfel, Hartmut, Dr., 51519 Odenthal (DE); Brockelt, Michael, 51379 Leverkusen (DE)

(56) Entgegenhaltungen:
- EP-A- 0 031 423
- EP-A- 0 161 600
- EP-A- 0 288 892
- DE-A- 1 568 087
- DE-A- 2 528 694

## Beschreibung

Die Erfindung betrifft ein Verfahren zur Fraktionierung und Reinigung von aromatischen Polyamingemischen und deren Verwendung.

Die Herstellung von aromatischen Polyaminen und Polyamingemischen, insbesondere der Diphenylmethanreihe, wird in zahlreichen Patentmeldungen und Patenten beschrieben, ebenso die Verwendung dieser Produkte. Herausragende Bedeutung kommt dabei der Verwendung dieser Produkte als Rohstoffe für die Herstellung von Isocyanaten zu, in der Regel durch Umsetzung der Polyamingemische mit Phosgen nach den allgemein üblichen und bekannten Methoden.

Die dabei resultierenden Isocyanate bzw. Isocyanatgemische fallen in vielen Fällen aber nicht in der Form und Zusammensetzung an, wie sie auf der Isocyanatstufe bevorzugt weiterverwendet werden, sondern müssen -vor durch teilweise aufwendige Aufarbeitungs- und Trennverfahren in die verwendungsgerechte Form übergeführt werden. Geeignete Polyaminvorstufen, die weniger aufwendig in die Isocyanatverwendungsformen gebracht werden können, sind in vielen Fällen verfahrenstechnisch schwierig oder gar nicht zugänglich oder wirtschaftlich unattraktiv herzustellen.

Beispielhaft ist die Gewinnung des für die Herstellung hochwertiger Polyurethanwerkstoffe wichtigen 4,4'-Diisocyanato-diphenylmethans, dessen Aminvorstufe in der Regel aus Anilin und Formaldehyd nur gemeinsam mit Isomeren, insbesondere dem 2,4'-Isomeren, und höherfunktionellen Polyaminen gewonnen werden kann. Diese Bestandteile sind zwar die Grundlage für ebenfalls begehrte Isocyanate, doch ist die Auftrennung der Rohisocyanate in die für die Weiterverwendung geeigneten Isocyanate bzw. Isocyanatgemische nicht einfach.

In der Regel werden zunächst ein Teil der Zweikernverbindungen von dem Rest abgetrennt. Anschließend wird aus der Zweikernfraktion in einem viele Trennstufen erfordernden zweiten Destillationsschritt das 4,4'-Diisocyanato-diphenylmethan von den anderen Isomeren befreit.

Das 2,4'-Isomere in angereicherter Form hat selbst in neuerer Zeit zunehmende Bedeutung als Polyurethanrohstoff erlangt, und kann nur mit beträchtlichem destillativen Aufwand gegenüber dem 4,4'-Isomeren angereichert und von dem gegebenenfalls vorhandenen 2,2'-Isomeren befreit werden.

Isomerentrennverfahren oder Anreicherungsverfahren innerhalb der Fraktion der höherkernigen Homologen bzw. der höherfunktionellen Bestandteile der Amine wie auch der Isocyanate der Diphenylmethanreihe sind praktisch nicht bekannt.

Zunehmendes Interesse findet auch das 4,4'-Diamino-diphenylmethan als Rohstoff für das Di-(4-isocyanatocyclohexyl)-methan, die kernhydrierte Form des 4,4'-Diisocyanato-diphenylmethans, wobei die Bereitstellung geeigneter aromatischer Polyamingemische für die Hydrierstufe mit einem möglichst hohen Gehalt an 4,4'-Diamino-diphenylmethan bei gleichzeitig einem möglichst geringen Anteil an 2,4'-Diamino-diphenylmethan sehr aufwendig ist.

Es ist bekannt, daß Amine durch partielle Überführung in ihre Salze in bestimmten Fällen getrennt werden können, wobei u.a. die unterschiedlichen Basenstärken genutzt werden. Dabei handelt es sich in der Regel um Monoamine mit stark unterschiedlichen Basenstärken.

Auch für aromatische Polyamingemische, insbesondere der Diphenylmethanreihe, sind solche Disproportionierungseffekte in zweiphasigen Systemen bereits beschrieben (DE-A 2238319 und DE-A 2528694).

Die Effekte sind infolge der in einem solchen Gemisch vorhanden zahlreichen Komponenten, deren Aminogruppen sich vom Typ her - praktisch alle sind Arylaminogruppen - kaum unterscheiden, nicht besonders groß und ausgeprägt, um für eine direkte Nutzung mit einfachen Mitteln interessant zu sein.

Diese Aufgabe konnte durch das erfindungsgemäße Verfahren, welches aufgrund der erfindungsgemäßen Ausführung überraschend hohe Trennleistung bei der Fraktionierung von aromatischen Polyamingemischen, insbesondere der Diphenylmethanreihe, erzielt, und dabei in der Wirkung weit über die bekannten Effekte des Standes der Technik hinausgeht, gelöst werden.

Bei der erfindungsgemäßen Fraktionierung von aromatischen Polyamingemischen werden andere, unterschiedlich zusammengesetzte Polyamingemische gewonnen.

Bei diesen abgeleiteten Polyamingemischen kann es sich um solche handeln, die auf bekannten Synthesewegen nur sehr aufwendig zugänglich sind. Dabei kann es sich auch um Polyamingemische handeln, die für eine vereinfachte Herstellung der Isocyanate besser geeignet sind, als die bekannten und technisch gut herzustellenden Polyamingemische, indem sie z.B. auf der Isocyanatstufe schwierig durchzuführende Isomerentrennungen auf der Aminstufe vorwegnehmen. Solche Gemische können auch völlig neuartige, weil nach dem Stand der Technik nicht darstellbare Polyamingemische sein, die zu völlig neuartigen Isocyanaten führen.

Andererseits kann das erfindungsgemäße Verfahren dazu genutzt werden, aus beliebigen, d.h. auch aus wiedergewonnenen Polyamingemischen, die sich durch Verunreinigung oder durch nichtstatistische, d.h. selektive Verluste bei einzelnen Komponenten bei der Wiedergewinnung von den ursprünglich eingesetzten Polyaminen oder Isocyanaten unterscheiden, Produktfraktionen zu gewinnen, welche dem Standard oder den Ausgangspolyaminen entsprechen.

Schließlich kann das erfindungsgemäße Verfahren dazu genutzt werden, synthesebedingte und im Endprodukt unerwünschte Neben- und Zwischenprodukte, mitzufraktionieren und in einer Produktfraktion ab - und in einer anderen entsprechend anzureichern, gegebenenfalls in einer eigenen Fraktion auszuschleusen.

Es handelt sich bei der vorliegenden Erfindung um ein breit anwendbares Verfahren, mit welchem die Aufgabe der Fraktionierung und Reinigung von aromatischen Di- und Polyamingemischen, insbesondere der Diphenylmethanreihe, gelöst werden kann.

Aufgabe war es, ein Verfahren zur Verfügung zu stellen, das es gestattet, aromatische Polyamingemische in einfacher Weise zu fraktionieren bzw. zu reinigen, so daß Isomere in reiner Form oder in angereicherter Form anfallen.

Gegenstand der Erfindung ist ein Verfahren zur Fraktionierung und Reinigung von aromatischen Polyamingemischen, insbesondere von Polyamingemischen der Diphenylmethanreihe, welches dadurch gekennzeichnet ist, daß man
a) das Polyaminausgangsgemisch (A) in einem zweiphasigen System, bestehend aus (i) einer hydrophoben Lösungsmittelphase (B), die im wesentlichen aus hydrophobem Lösungsmittel und gegebenenfalls einem aromatischen Hilfsamin, welches in Wasser praktisch unlöslich ist und unter Normaldruck einen mindestens 20°C unter dem Siedepunkt der am niedrigsten siedenden Komponente des Ausgangsgemisches und mindestens 20°C oberhalb des Siedepunktes des Lösungsmittels liegenden Siedepunkt aufweist, und/oder gegebenenfalls Polyaminen besteht, und (ii) einer wäßrigen Phase (C), bestehend im wesentlichen aus Wasser, einer starken Säure und gegebenenfalls zumindest teilweise in der Salzform vorliegendem Hilfsamin, sowie gegebenenfalls zumindest teilweise in der Salzform vorliegenden Polyaminen unter Zuhilfenahme einer nach dem Gegenstromprinzip arbeitenden Extraktionsstufe (6) unter Durchmischung der Phasen verteilt, indem man das Ausgangspolyamingemisch vorzugsweise über die organische Phase (B) in die Extraktionsstufe (6) einbringt, mit der Maßgabe, daß in diesem zweiphasigen System die Summe der in den Mengenströmen (A), (B) und (C) eingebrachten Aminäquivalente die Anzahl der im Mengenstrom (C) eingebrachten Säureäquivalente stets übersteigt, die diese Extraktionsstufe verlassende organische Phase (D) zumindest teilweise, gegebenenfalls nach Durchlaufen einer Waschstufe (10), in einer im Falle der bevorzugten Mitverwendung von Hilfsamin, vorzugsweise mehrstufig durchgeführten Destillationsstufe (11), in eine erste, in der Extraktionsstufe (6) wiederverwendete Fraktion (E), bestehend im wesentlichen aus hydrophobem Lösungsmittel und gegebenenfalls Hilfsamin, gegebenenfalls eine zweite Fraktion (F), bestehend im wesentlichen aus Hilfsamin und gegebenenfalls hydrophobem Lösungsmittel und eine als Destillationsrückstand (G) anfallende erste Polyaminfraktion auftrennt, und die die erste Extraktionsstufe (6) verlassende wäßrige Phase (H)
b) gegebenenfalls zumindest teilweise über eine nachgeschaltete Extraktionsstufe (7)
c) in eine Neutralisationsstufe (8) leitet, mit Basen, vorzugweise wäßriger Natronlauge, die in der wäßrigen Phase enthaltene Säure neutralisiert und anschließend in einem Phasentrennschritt in eine wäßrige Phase, enthaltend die Säure in Form ihrer neutralen Salze und eine organische Phase, enthaltend im wesentlichen Polyamin, gegebenenfalls Hilfsamin und gegebenenfalls geringe Mengen hydrophoben Lösungsmittels, mechanisch auftrennt und
d) die in der Neutralisationsstufe (8) anfallende organische Phase (J) gegebenenfalls nach Durchlaufen einer Waschsstufe (9), zumindest teilweise in einer Destillationsstufe (12) aufarbeitet in eine Destillationsfraktion (K), enthaltend die in (J) bzw. (J') enthaltenen Anteile an hydrophobem Lösungsmittel und das gegebenenfalls in (J) bzw. (J') enthaltene Hilfsamin, und in eine als Destillationsrückstand (L) anfallende zweite Polyaminfraktion.

Bevorzugt wird das Verfahren so durchgeführt, daß man
b) die in der Extraktionsstufe (6) anfallende wäßrige Phase (H) zumindest teilweise in einer nachgeschalteten, nach dem Gegenstromprinzip arbeitenden Extraktionsstufe (7) extrahiert unter Verwendung einer organischen Phase (O) als Extraktionsmittel, bestehend neben hydrophobem Lösungsmittel aus Hilfsamin und/oder Polyamin, letzteres vorzugsweise mit der Zusammensetzung des zweiten Teilproduktes (L) und vorzugsweise eingebracht als Teilmenge (J'') des Mengenstroms (J), die in Verfahrensstufe (7) resultierende organische Phase (M) dem Mengenstrom (B) zuschlägt, und damit der Extraktionsstufe (6) zuführt und die in (7) resultierende wäßrige Phase (N) der Neutralisationsstufe (8) zuführt.

Das erfindungsgemäße Verfahren wird besonders bevorzugt so durchgeführt, daß man die die Extraktionsstufe (6) verlassende organische Phase zumindest teilweise in einer (6) vorgelagerten Stufe (6A) mit der wäßrigen Säure (Mengenstrom X) und gegebenenfalls Wasser (Mengenstrom Y) und/oder gegebenenfalls Hilfsamin vermischt, und von dem resultierenden Gemisch gegebenenfalls nach Auftrennung in eine organische und eine wäßrige Phase wenigstens die wäßrige Phase der Verfahrensstufe (6) und die gegebenenfalls abgetrennte organische Phase dem organischen Mengenstrom (D) zuführt.

Eine weiter verbesserte und daher bevorzugte Ausführungsform des erfindungsgemäßen Verfahrens, wird dadurch erreicht, daß man eine Teilmenge (D'') der die Extraktionsstufe (6) verlassenden organischen Phase (D) abtrennt und in einer gegebenenfalls mehrstufigen, in der ersten Stufe vorzugsweise als Mischer-Scheidereinheit betriebenen Extraktionsstufe (5) mit zumindest einer Teilmenge, vorzugsweise mit dem gesamten Mengenstrom (X) der wäßrigen Säure im Gegenstrom extrahiert, gegebenenfalls unter Zugabe von Hilfsamin, und den Teilmengenstrom (D'') so bemißt, daß dabei ein möglichst weitgehender Übergang des in (D'') enthaltenen Polyamins in die die Extraktionseinheit (5) verlassende wäßrige Phase (Q) stattfindet, besagte wäßrige Phase (Q) anteilig oder insgesamt direkt und/oder über den Mischer (6A), gegebenenfalls nach Zugabe von Wasser aus Mengenstrom (Y) und/oder Hilfsamin und/oder weiterer wäßriger Säure als wäßrige Phase (C) der Extraktionsstufe (6) zuführt, die in (5) anfallende organische Phase (P), bestehend im wesentlichen aus hydrophobem Lösungsmittel und gegebenenfalls Hilfsamin, ebenfalls der der Extraktionsstufe (6) zugeführten organischen Phase (B) zuschlägt und als Lösungsmittel für das Ausgangspolyamin (A) verwendet.

Als Hilfsamin wird vorzugsweise Anilin eingesetzt und als Polyamingemisch der Diphenylmethanreihe bevorzugt ein Polyamingemisch, wie es bei der säurekatalysierten Anilin-/Formaldehyd-Kondensation anfällt.

Die derart behandelten Polyamingemische, also die mit dem erfindungsgemäßen Verfahren erzeugten Fraktionen werden zur Herstellung der entsprechenden aromatischen Polyisocyanatgemische und zur Herstellung von Polyurethankunststoffen verwendet.

Außerdem können die nach dem erfindungsgemäßen Verfahren erzeugten Fraktionen zur Herstellung der entsprechenden kernhydrierten Polyamine oder als Vernetzer und als Epoxidhärter verwendet werden.

Die aus den fraktionierten Polyamingemischen hergestellten entsprechenden Polyisocyanate werden bevorzugt zur Herstellung von PU-Schaumstoffen eingesetzt.

Ausgangsgemische sind beispielsweise technische Arylamingemische, wie sie bei der Herstellung aus den Ausgangsverbindungen oder wie sie bei der Wiedergewinnung anfallen.

Beispiele von Ausgangsarylamingemischen, für deren Fraktionierung und Reinigung das erfindungsgemäße Verfahren besonders geeignet ist, sind
1. Polyamingemische der Diphenylmethanreihe, wie sie bei der Kondensation und säurekatälysierten Umlagerung von Anilin mit Formaldehyd entstehen,
2. Polyamingemische der Diphenylmethanreihe, wie sie bei der säurekatalysierten Kondensation von substituierten Anilinen mit Formaldehyd anfallen,
3. Polyamingemische der Diphenylmethanreihe, wie sie bei der Mischkondensation von substituierten Anilinen untereinander und/oder mit Anilin mit Formaldehyd anfallen,
4. Polyamingemische der Diphenylmethanreihe, wie sie bei der Kondensation, auch der Mischkondensation von substituierten Anilinen und/oder Anilin mit Aldehyden und/oder Ketonen anfallen,
5. Polyamingemische der Diphenylmethanreihe, wie sie bei der Nitrierung und anschließenden Reduktion von Di- und/oder Polyarylmethanen und/oder substituierten Di- und/oder Polyarylmethanen entstehen; unter Polyarylmethanen werden hier insbesondere die Benzylhomologen des Diphenylmethans verstanden,
6. Polyamingemische der Diphenylmethanreihe, wie sie bei der Kondensation von Monoarylmonoaminen (z.B. Anilin, substituierte Aniline) und/oder Monoaryldiaminen (Phenylendiamine, substituierte Phenylendiamine) mit Aldehyden, Ketonen, insbesondere Formaldehyd, und säurekatalysierter Umlagerung entstehen und
7. Polyamingemische der Triphenylmethanreihe, wie sie z.B.bei der Nitrierung und anschließenden Reduktion von Triphenylmethan, insbesondere alkylsubstituierten Triphenylmethanen und seinen höherkernigen, insbesondere Benzylhomologen entstehen.

Bei den zum Einsatz gelangenden hydrophoben Lösungsmitteln handelt es sich um inerte Lösungsmittel des Siedepunktbereiches von 30° - 280°C, vorzugsweise von 80° - 200°C, wie beispielsweise Chlorbenzol, Dichlorbenzol, Benzol, Toluol, Ethylbenzol, Cumol, Xylol, Dichlorethan, Chloroform und Tetrachlorkohlenstoff. Vorzugsweise werden Xylole, d.h. technische Xylolgemische, insbesondere o-Xylol, Toluol, Ethylbenzol, Cumol und Chlorbenzol eingesetzt.

Bevorzugt werden solche Lösungsmittel verwendet, die ein gutes Lösungsvermögen für die eingesetzten Polyamingemische aufweisen.

Bei den eingesetzten Säuren handelt es sich um wasserlösliche Protonsäuren mit einem unter 2,5 , vorzugsweise unter 1,5 liegenden pKA-Wert. Beispiele hierfür sind Salzsäure, Bromwasserstoffsäure, Schwefelsäure, Trifluoressigsäure, Methansulfonsäure oder Phosphorsäure. Bevorzugt eingesetzt werden Salzsäure und Schwefelsäure.

Die genannten Säuren können auch im Gemisch mit sauren oder neutralen Salzen derartiger Säuren, wie z.B. den entsprechenden Ammoniumsalzen oder auch den entsprechenden Alkalisalzen eingesetzt werden.

Im allgemeinen liegen die genannten Säuren in der wäßrigen Phase (C) vor entweder als wäßrige Lösung der freien Säure oder als wäßrige Lösung, die neben der freien Säure auch die Ammoniumsalze der Säure mit Hilfsamin und/oder Polyamin enthalt oder als wäßrige Lösung, in der die Säure vollständig in der Form ihrer Aminoniumsalze mit Hilfsamin und/oder Polyamin vorliegt und die gegebenenfalls noch weiteres, nicht salzartig gebundenes Hilfsamin enthält.

Spätestens nach Durchlaufen der Extraktionsstufe (6) liegen die genannten Säuren in der wäßrigen Phase in der Form der Aminoniumsalze der Säure mit der in der wäßrigen Phase befindlichen Fraktion des Polyamins und gegebenenfalls mit Hilfsamin vor.

Nach Durchlaufen der Extraktionsstufe, gegebenenfalls der Extraktionsstufen, wird die in der wäßrigen Phase befindliche Säure durch Neutralisation mit starken Basen in die entsprechenden Neutralsalze übergeführt. Dabei werden die salzartig gebundenen Polyamine und gegebenenfalls Hilfsamin freigesetzt.

Als Hilfsamin finden in der Regel Verwendung Monoarylamine oder Gemische von Monoarylaminen wie z.B. Anilin und/oder am Kern und/oder am Stickstoff alkylsubstituierte Anilinderivate.

Bevorzugt werden primäre Aniline, besonders bevorzugt sind neben Anilin das 2,6-Dimethylanilin, Xylidingemische und 2-Methyl-6-ethylanilin.

Das erfindungsgemäße Verfahren kann sowohl diskontinuierlich als auch kontinuierlich durchgeführt werden. Bevorzugte Ausführungsform ist die kontinuierliche Arbeitsweise. Dabei wird das Verfahren in allen Stufen unter dem Eigendruck des Systems und vorzugsweise in einer Inertgasatmosphäre (Stickstoff) durchgeführt.

Das erfindungsgemäße Verfahren kann sowohl mit einer (Abb.1.1 und Abb. 1.2) als auch mit zwei (Abb. 2 und Abb.3) oder drei (Abb.4) Extraktionsstufen durchgeführt werden.

Das erfindungsgemäße Verfahren kann zur Steigerung des Anreicherungs- bzw. korrespondierenden Abreicherungseffektes mit jeder der anfallenden Produktfraktionen wiederholt werden.

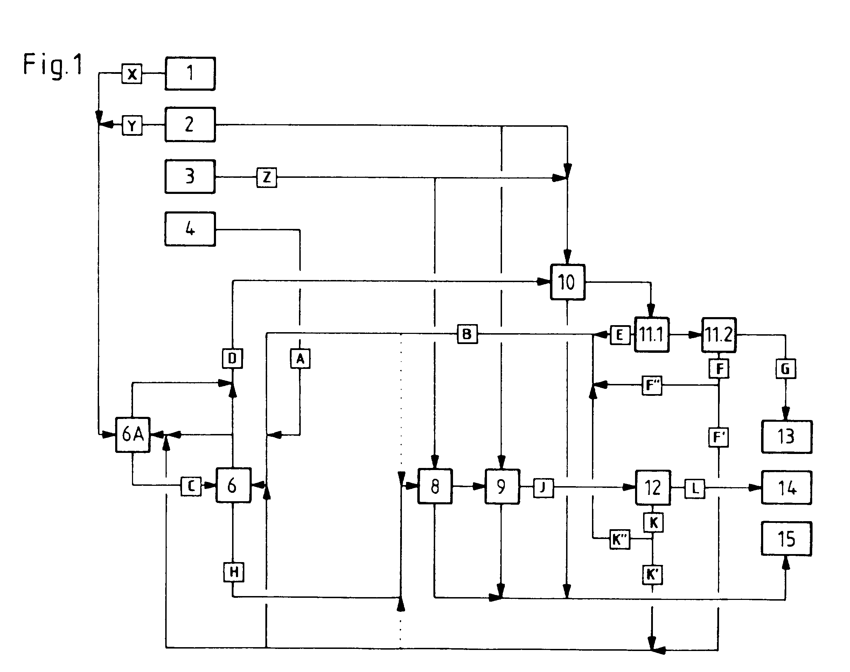

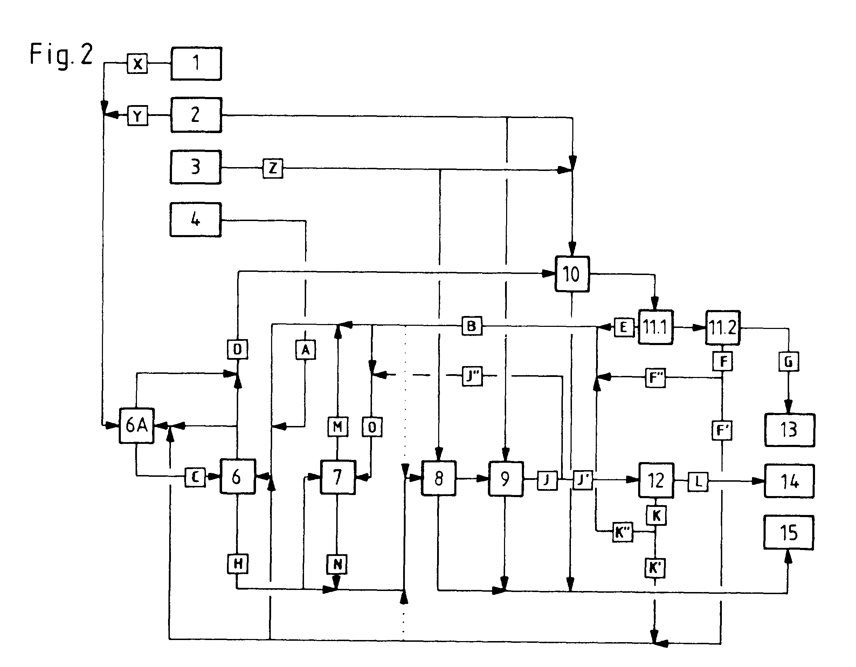

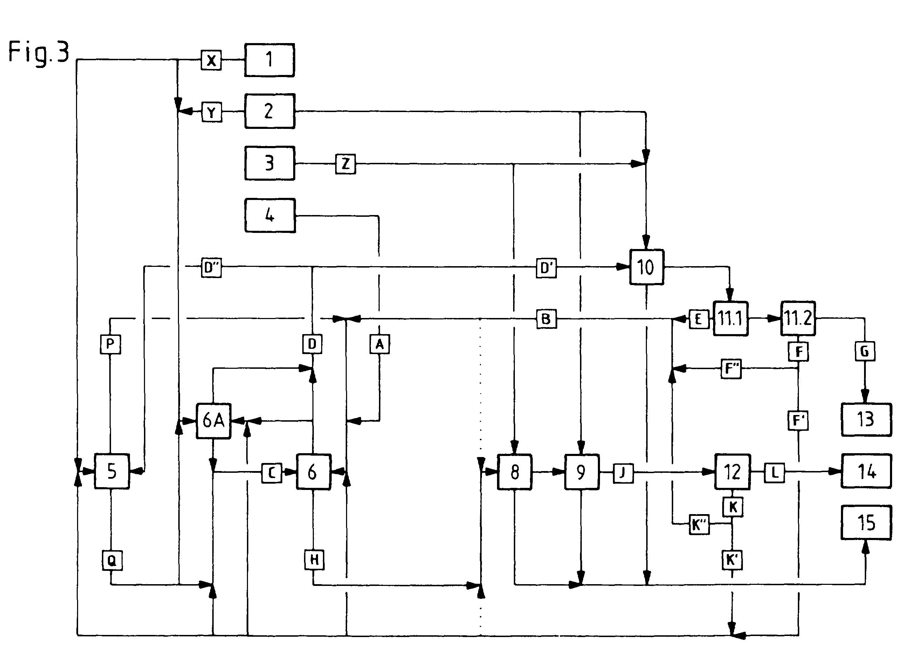

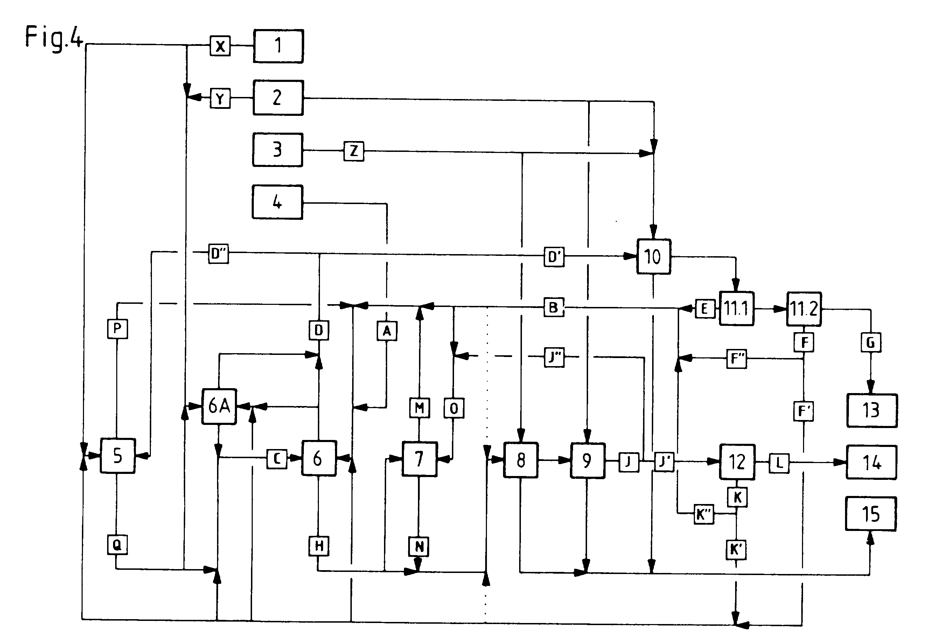

Die in Abb.1 bis 4 dargestellten Fließdiagramme dienen der weiteren Erläuterung des erfindungsgemäßen Verfahrens. In diesen Abbildungen bedeuten :
(1) einen Tank für wäßrige Säure
(2) einen Tank für Wasser
(3) einen Tank für wäßrige Base
(4) einen Tank für Ausgangspolyamin
(5) einen ein- oder mehrstufigen Extraktor, dessen aus der Sicht der wäßrigen Phase erste Stufe in der Regel aus einer Mischer-Scheidereinheit besteht
(6A) einen Mischer oder eine Mischer-Scheidereinheit
(6) eine (erste) Extraktionsstufe
(7) eine (zweite) Extraktionsstufe
(8) eine Neutralisationsstufe
(9) eine Waschstufe
(10) eine Waschstufe
(11.1) eine erste Destillationsstufe einer gegebenenfalls Mehrstufendestillation
(11.2) eine letzte Destillationsstufe einer gegebenenfalls Mehrstufendestillation
(12) eine Destillationsstufe
(13) einen Tank für ein erstes Verfahrensprodukt
(14) einen Tank für ein weiteres Verfahrensprodukt
(15) einen Tank für Abwasser

Die Bezugszeichen A - Q, X, Y und Z bezeichnen die Mengenströme, auf die nachstehend und in den Beispielen Bezug genommen wird.

Bei der Extraktionsstufe (5) handelt es sich im einfachsten Fall um eine einstufig wirkende Mischer-Scheidereinheit, vorzugsweise kommen jedoch mehrstufig wirkende Extraktionseinheiten zum Einsatz, wobei die aus Sicht des Mengenstromes (X) erste Stufe in der Regel aus einer Mischer-Scheidereinheit besteht.

Bei der Stufe (6A) handelt es sich im einfachsten Fall um einen Mischer oder eine Mischer-Scheidereinheit, deren Scheiderteil gegebenenfalls in den nachfolgenden Extraktor (6) integriert ist.

Bei der Extraktionsstufe (6) handelt es sich im einfachsten Fall um eine einstufig wirkende Mischer-Scheidereinheit, vorzugsweise kommen jedoch mehrstufig wirkende Extraktionseinheiten zum Einsatz.

Die gegebenenfalls nachgeschaltete Extraktionsstufe (7) besteht im einfachsten Falle ebenfalls aus einer Mischer-Scheidereinheit, vorzugsweise kommen jedoch auch hier mehrstufig wirkende Extraktionseinheiten zum Einsatz.

Die mehrstufig wirkenden Extraktionseinheiten können aus mehreren in Serie geschalteten Extraktoren bestehen. Vorzugsweise werden die üblichen Gegenstromextraktionsvorrichtungen verwendet.

Bei der Neutralisationsstufe (8) handelt es sich um eine Vorrichtung zur intensiven Durchmischung der wäßrigen Phasen (H) bzw. (N) zur Umsetzung der enthaltenen Säure mit der wäßrigen Lösung einer starken Base (Z) im Überschuß aus Behälter (3) mit der Möglichkeit, Neutralisationswärme abzuführen, und anschließender Abtrennung des Polyamins.

Zum Durchmischen werden im einfachsten Falle ein oder mehrere gerührte Kessel eingesetzt, dabei kann der Mischvorgang durch Mischdüsen, Intensivmischer und/oder Umpumpvorrichtungen verstärkt werden. Für die anschließende Phasentrennung werden im einfachsten Falle Scheider eingesetzt, wobei die Phasentrennung durch den Einbau von Scheidehilfen verstärkt werden kann. Ebenso geeignet sind beispielsweise Zentrifugen.

In den Fällen, in denen nach der Umsetzung der wäßrigen Phasen (H) bzw. (N) mit starken Basen die einfache mechanische Abtrennung schwierig oder nicht möglich ist, wird die Abtrennung durch den Einsatz von zusätzlichen hydrophobem Lösungsmittel und/oder Hilfsamin durchgeführt, gegebenenfalls als Extraktionsvorgang in einen vorzugsweise mehrstufig wirkenden Extrator.

Bei der Waschstufe (9), handelt es sich im einfachsten Fall um eine Mischer-Scheidereinheit, in welcher der Mengenstrom (J) mit Wasser gewaschen wird, für die Durchführung des erfindungsgemäßen Verfahrens ist die Waschstufe (9) grundsätzlich nicht erforderlich, in der Regel aber vorteilhaft.

Auch bei der Waschstufe (10) handelt es sich im einfachsten Fall um eine Mischer-Scheidereinheit, vorzugsweise kommen jedoch mehrstufig wirkende Extraktoren zum Einsatz.

Die Waschstufe (10) kann sowohl mit Wasser als auch vorzugsweise mit verdünnten wäßrigen Lösungen starker Basen durchgeführt werden.

Die Destillationsstufe (11) besteht im einfachsten Fall aus einer Destillationskolonne, mit welcher das Zulaufprodukt (D) bzw. (D') in eine Destillatfraktion (E), enthaltend das hydrophobe Lösungsmittel und gegebenenfalls vorhandenes Hilfsamin, und den Destillationsrückstand (G), bestehend aus einer ersten Polyaminfraktion, aufgetrennt wird.

Bei der Mitverwendung von Hilfsamin besteht die Destillationsstufe (11) vorzugsweise aus einer wenigstens zweistufigen Mehrstufendestillation, deren erste Stufe (11.1) ein gegenüber dem Zulaufprodukt (D) bzw. (D') von Polyamin befreites und an Hilfsamin verarmtes hydrophobes Lösungsmittel als Destillat (E) liefert, und deren letzte Stufe (11.2) ein gegenüber (D) von Polyamin befreites und an Lösungsmittel verarmtes Hilfsamin als Destillat (F) liefert.

Zusätzlich fällt bei der letzten Destillationsstufe (11.2) die in Mengenstrom (D) enthaltene erste Fraktion des Ausgangspolyamin (A) als Destillationsrückstand (G) an.

Bei der Mitverwendung von Hilfsamin ist die vollständige destillative Auftrennung von hydrophobem Lösungsmittel und Hilfsamin bei Durchführung des erfindungsgemäßen Verfahrens nicht erforderlich.

Die Destillationsstufe (12) besteht im einfachsten Fall aus einer Destillationskolonne. Bei der Durchführung des erfindungsgemäßen Verfahrens ohne Mitverwendung eines Hilfsamins enthält das Zulaufprodukt (J) bzw. (J') im einfachsten Fall nur geringe Mengen an hydrophobem Lösungsmittel. Bei Zugabe von hydrophobem Lösungsmittel und/oder Hilfsamin vor oder während der Neutralisationsstufe müssen diese jedoch in (12) wieder abgetrennt werden.

Bei Durchführung der Neutralisationsstufe (8) unter Mitverwendung von zusätzlichem Hilfsamin enthält das Zulaufprodukt zur Destillationsstufe (12) in der Regel beträchtliche Anteile an Hilfsamin und nur geringe Anteile an hydrophobem Lösungsmittel, die im allgemeinen gemeinsam als Destillatfraktion (K) von der als Destillationsrückstand anfallenden zweiten Polyaminfraktion (L) abgetrennt werden.

Erfordert die Durchführung der Neutralisationsstufe (8), auch bei der Mitverwendung von Hilfsamin, den Einsatz von hydrophobem Lösungsmittel in (8) und/oder (9), beispielsweise zur Verbesserung der Neutralisationsreaktion oder zur Erleichterung der anschließenden Phasentrennung, dann wird auch die Destillationsstufe (12) vorzugsweise wenigstens zweistufig ausgeführt zur Gewinnung eines weitgehend an hydrophobem Lösungsmittel verarmten Destillates (K) in der letzten Stufe (12.2).

Auch aus Gründen einer besseren Energienutzung werden die Destillationsstufen (11) und (12) vorzugsweise mehrstufig durchgeführt.

Das erfindungsgemäße Verfahren kann sowohl unter Verwendung von hydrophobem Lösungsmittel allein als auch unter Verwendung von hydrophobem Lösungsmittel und Hilfsamin durchgeführt werden.

Das erfindungsgemäße Verfahren kann in mehreren technischen Varianten durchgeführt werden.

Gemäß einer ersten Variante erfolgt die Einspeisung des Ausgangspolyamingemisches (Mengenstrom A) aus Behälter (4) durch Vermischen mit Mengenstrom (B), bestehend aus hydrophobem Lösungsmittel und gegebenenfalls Hilfsamin.

Nach Zugabe von Ausgangspolyamin (A) zu Mengenstrom (B) liegt der Gehalt an Arylamin, bestehend aus Polyamin und Hilfsamin, im allgemeinen bei 5 - 90 Gew.-%, vorzugsweise bei 10 - 60 %. Der Gehalt an Ausgangspolyamin liegt im allgemeinen bei 5 - 90 Gew.-%, vorzugsweise bei 10 - 60 Gew.-%.

Die Einspeisung der Säure (Mengenstrom X) erfolgt über die wäßrige Phase (C).

Im allgemeinen besteht der Mengenstrom (C) aus Wasser, einer starken Protonsäure und gegebenenfalls Hilfsamin und/oder gegebenenfalls Polyamin.

Im allgemeinen liegt die Säure in der wäßrigen Phase (C) vor als wäßrige Lösung der Säure, die gegebenenfalls Ammoniumsalze der Säure mit Hilfsamin und/oder Polyamin enthält; vorzugsweise liegt die Säure vor als wäßrige Lösung ihrer Ammnoniumsalze mit Hilfsamin und/oder Polyamin, die gegebenenfalls freies, d.h. nicht salzartig gebundenes Hilfsamin und/oder Polyamin gelöst enthält.

Bei einer bevorzugten Ausführungsform, wird der Verfahrensstufe (6A) außer der wäßrigen Säure (X) und gegebenenfalls zusätzlichem Wasser (Y) gegebenenfalls Hilfsamin und/oder vorzugsweise die die Extraktionsstufe (6) verlassende organische Phase zumindest in solcher Menge zugeführt, daß sich das gewünschte Amin-Säureverhältnis in der die Stufe (6A) verlassenden wäßrigen Phase einstellt.

Es ist für das erfindungsgemäße Verfahren nicht von ausschlaggebender Bedeutung, ob im Falle der Bildung eines zweiphasigen Gemisches im Mischer der Verfahrensstufe (6A) dieses als solches ohne vorherige Phasentrennung der Extraktionsstufe (6) in die aus Sicht der wäßrigen Phase erste Stufe zugeführt wird (Abb: 1.1), oder ob die Stufe (6A) im Falle der Bildung eines zweiphasigen Gemisches als Mischer-Scheidereinheit ausgelegt und nur die abgetrennte wäßrige Phase nach (6) geführt und die abgetrennte organische Phase dem Mengenstrom (D) zugeschlagen wird (Abb. 1.2); wenngleich dieser zweite Fall bevorzugt ist insbesondere bei zunehmender Menge an organischer Phase.

Es hat sich als zweckmäßig erwiesen, den Säuregehalt der wäßrigen Phase unabhängig von dem sich je nach Verfahrensparametern (z.B. Zusammensetzung von organischer und wäßriger Phase, Phasenverhältnis, Temperatur) in der wäßrigen Phase eines zweiphasigen Systems einstellenden Amingehalt und über eine sogenannte "Molarität" zu definieren.

Die "Molarität" wird festgelegt als theoretische Konzentration von zu 100 % protoniertem Amin (d.h. gleiche Anzahl von Säure- und Aminäquivalenten) in einem rechnerisch um den Anteil an nicht protonierten Amin vermindertem Volumen oder gegebenenfalls in einem rechnerisch um eine entsprechende Aminmenge bis zur vollständigen Bindung der Säure als Ammoniumsalze erweitertem Volumen an wäßriger Phase.

Die so definierte Molarität kann Werte bis 6 annehmen und wird je nach der der jeweiligen Ausführungsform zugrunde liegenden - hier produktbezogenen - Trennaufgabe in diesem Bereich gezielt variiert.

Der für die jeweilige Ausführungsform des erfindungsgemäßen Verfahrens wohldefinierte und in engen Grenzen gemessene und geregelte Säuregehalt des Mengenstroms (C) wird als wichtige Führungsgröße je nach der der jeweiligen Ausführungsform zugrunde liegenden - hier produktbezogenen - Trennaufgabe in einem weiten Bereich insgesamt oder in einzelnen Verfahrensstufen gezielt variiert, gegebenenfalls unter Zufuhr von Wasser aus Mengenstrom (Y) oder wäßriger Säure aus Mengenstrom (X).

Nach oben wird dieser Arbeitsbereich praktisch begrenzt einerseits durch zunehniende Kristallisationsneigung der Aminsalze mit zunehmender Konzentration, insbesondere bei hohen Protonierungsgraden, und andererseits durch die zunehmende gegenseitige Löslichkeit der Phasen ineinander, insbesondere bei niedrigen Protonierungsgraden.

Der Protonierungsgrad gibt das Verhältnis von Säureäquivalenten zu Aminäquivalenten wieder.

Nach unten wird dieser Bereich wirtschaftlich begrenzt durch den abnehmenden Säuregehalt und damit die quantitative Abnahme der Trennleistung, d.h. bei hervorragender qualitativer Trennleistung und technisch problemlos, ist mit sinkender Molarität ein zunehmend größeres Volumen an wäßriger Phase erforderlich für die Trennung einer gegebenen Aminmenge.

In der vorzugsweise mehrstufig betriebenen Extraktionsstufe (6) werden die organische Phase (B) und die wäßrige Phase (C) unter inniger Durchmischung einander entgegengeführt.

Bei diesem Vorgang findet in der Regel ein Übergang von Polyarylamin von der organischen Phase (B) in die wäßrige Phase (C) statt, gegebenenfalls im Austausch gegen Arylamin in entgegengesetzter Richtung.

In der die Extraktionsstufe (6) verlassenden wäßrigen Phase (H) liegt die Säure als wäßrige Lösung ihrer Ammoniumsalze mit Polyamin und gegebenenfalls Hilfsamin vor, die in der Regel freies, d.h. nicht salzartig gebundenes Polyamin und gegebenenfalls freies, d.h. nicht salzartig gebundenes Hilfsamin gelöst enthält.

Das zusammen mit der organischen Phase (B) in den Extraktor (6) eingebrachte Ausgangspolyamin (A) verteilt sich auf die den Extraktor verlassende wäßrige Phase (H) und die den Extraktor (6) verlassende organische Phase (D) (quantitative Fraktionierung).

Die mengenmäßige Aufteilung der einzelnen Komponenten des Ausgangspolyamingemisches auf die resultierende wäßrige Phase (H) und die resultierende organische Phase (D) erfolgt unter den Bedingungen des erfindungsgemäßen Verfahrens mit einer überraschend hohen Selektivität, so daß die resultierenden Produktfraktionen eine andere, unter Umständen stark von der des Ausgangspolyamingemisches abweichende Zusammensetzung aufweisen (qualitative Fraktionierung).

Beispielsweise ausgehend von den bevorzugt eingesetzten Anilinformaldehydkondensationsprodukten wurde gefunden, daß von einer in zwei oder mehreren isomeren Formen im Ausgangsgemisch enthaltenen Polyaminkomponente in der Regel die orthoisomere(n) Form(en) in der die Trennstufe (6) verlassenden organischen Phase (D) relativ angereichert ist (sind); beispielsweise 2.4'-Diamino-diphenylmethan relativ zu 4,4'-Diamino-diphenylmethan. Umgekehrt ist die resultierende wäßrige Phase (H) relativ verarmt an dem 2,4-'Isomeren, während das 4,4'-Isomere relativ angereichert ist.

Sind mehrere "ortho-Isomere" im Ausgangspolyamin vorhanden, z.B. 2,2'- und 2,4'-Diamino-diphenylmethan, dann ist das "ortho-reichere" 2,2'-Isomere in der organischen Phase (D) gegenüber dem "ortho-ärmeren" 2,4'-Isomeren stärker angereichert, welchletzteres seinerseits gegenüber dem "noch ortho-ärmeren" 4,4'-Isomeren relativ angereichert ist.

Der zuerst bei den Anilinformaldehydkondensationsprodukten der Diamino-diphenylmethanreihe gefundene An- und Abreicherungseffekt wird rein empirischdeskriptiv mit dem Kriterium der ortho- und para-Substitution verbunden. Die davon abgeleitete Charakterisierung der Verfahrensprodukte als "orthoreich" und "orthoarm" ist dabei relativ und wird durch den Begriff "ortho-Substitutionsgrad" ausgedrückt.

Als "ortho-Substitutionsgrad" wird dabei das Verhältnis der orthoständigen Aminogruppen-Methylengruppenrelationen, zur Gesamtzahl aller Aminogruppen-Methylengruppenreiationen definiert. Mit diesem Begriff lassen sich praktisch alle Isomerentrennungen bei den Polyaminen erfassen, die aus Arylaminen, auch substituierten, mit Carbonylverbindungen in wäßrig saurem Medium hergestellt werden.

Überraschenderweise wurde nun der gleiche An- und Abreicherungseffekt - geordnet nach ortho-Substitutionsgrad - auch für die gut charakterisierten und analytisch erfaßbaren isomeren Dreikernverbindungen aus der Anilin-Formaldehydkondensation gefunden.

Analoges gilt für die Trennung der Isomeren von Kondensationsprodukten aus Formaldehyd mit Anilin und Diaminoarylverbindungen wie Phenylendiamin oder alkylsubstituierten Phenylendiaminen.

Die bisher erwähnten Polyamingemische weisen, bedingt durch ihre Herstellung, Aminogruppen auf, die praktisch nur orthoständig und/oder paraständig zu Methylengruppen sind. Dabei werden innerhalb einer Gruppe isomerer Verbindungen in der Regel diejenigen mit dem höheren ortho-Substitutionsgrad gegenüber den Isomeren mit einem geringeren ortho-Substitutionsgrad bei der Fraktionierung in der organischen Phase (D) angereichert.

Polyamingemische insbesondere der Diphenylmethanreihe einschließlich der jeweiligen höherkernigen Homologen, die nach anderen Verfahren hergestellt werden, beispielsweise durch Nitrierung von Diphenylmethan oder Methyldiphenylmethanen und anschließende Reduktion weisen außer ortho- und para-ständigen Aminogruppen herstellungsbedingt auch andere Aminogruppen-Methylengruppenrelationen auf. Für diese Polyamingemische ist das erfindungsgemäße Verfahren genauso wirksam.

Beispielsweise läßt sich aus einem Gemisch von 2- und 4-Methyldiphenylmethan durch Nitrierung und anschließende Reduktion ein Polyamingemisch herstellen, welches in der Hauptsache ein Isomerengemisch darstellt aus 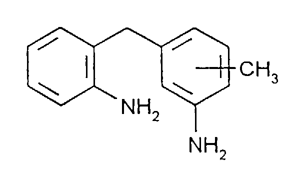 und

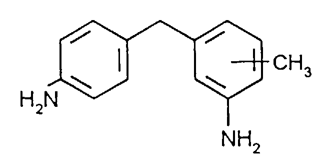

Bei der Fraktionierung solcher Gemische mit Hilfe des erfindungsgemäßen Verfahrens werden die 3,2'-Amino-Isomeren in der organischen Phase (D) gegenüber den 3,4'-Amino-Isomeren angereichert.

Das Kriterium "orthoreich" und "orthoarm" oder der "ortho-Substitutionsgrad" erfaßt in diesen Polyamingemischen nicht mehr alle Isomeren und ist daher sinngemäß anzuwenden, indem anstelle der Begriffe "orthoständig" und "paraständig" eine Einteilung der Isomeren vorgenommen wird in solche mit kleinerem (ortho-) und solche mit größerem ( para-) räumlichen Abstand der - in der Regel an unterschiedlichen Sechsringen befindlichen - Aminogruppen zur Methylenbrücke bzw. der Aminogruppen zueinander.

Eine weitere Klasse aromatischer Polyamingemische, die sich mit Hilfe des erfindungsgemäßen Verfahrens sehr wirksam fraktionieren lassen, stellen die Polyamine des Triphenylmethans und seiner höherkernigen Homologen, vorzugsweise Benylhomologen dar, wie sie z.B. durch Nitrierung und anschließende Reduktion der entsprechenden Kohlenwasserstoffgemische hergestellt werden.

Bei der Fraktionierung technischer Polyamingemische der zuletzt genannten Substanzklassen
I. Mischkondensationsprodukte von Mono und Diaminoarylverbindungen mit Formaldehyd bzw. allgemeinen Carbonylverbindungen,
II. Polyamingemische aus Verfahren durch Nitrierung und anschließende Reduktion von Diphenylmethan und vorzugsweise substituierten insbesondere alkylsubstituierte Diphenylmethanen und den jeweiligen Homologen und
III. Polyamingemische aus Verfahren durch Nitrierung und anschließende Reduktion von Triphenylmethan und vorzugsweise substituierten insbesondere alkylsubstituierten Triphenylmethanen und den jeweiligen höherhernigen Benzylhomologen
wurde zusätzlich zur reinen Isomerentrennung eine weitere überrraschende Selektivität gefunden.

Polyamingemische der genannten Substanzklasse I bis III enthalten oder können enthalten Komponenten, bei denen wenigstens ein Arylkern pro Molekül mehr als eine, in der Regel zwei Aminogruppen trägt. Diese Komponenten können die bevorzugten Bestandteile des Polyamingemisches sein, ohne daß sie verfahrensbedingt mengenmäßig die Hauptprodukte sein müssen.

Zur besseren Charakterisierung solcher Komponenten wird der Begriff "Aminosubstitutionsgrad" verwendet, mit dem in erster Linie die Anzahl der Aminogruppen einer Komponente im Verhältnis zur Anzahl der Arylkerne gekennzeichnet wird.

Für Anilin und seine Kondensationsprodukte mit Formaldehyd ist dieser Ausdruck stets 1,0, für Phenylendiamin und seine Kondensationsprodukte stets 2,0. Für reine Mischkondensate ergeben sich für die Diphenylmethanisomeren der Wert 1,5 und für die höherkernigen Homologen Werte zwischen >1,0 und <2,0. Bei statistischer Verwendung des Begriffes Aminosubstitutionsgrad zur Charakterisierung von technischen Polyamingemischen ergeben sich ebenfalls Werte zwischen 1,0 und 2,0.

Bei der Fraktionierung von Polyamingemischen mit einem Aminosubstitutionsgrad >1,0 wurde nun gefunden, daß die Komponenten mit einem höheren Aminosubstitutionsgrad in der resultierenden wäßrigen Phase (H) relativ angereichert werden, und zwar um so stärker je größer der Aminosubstitutionsgrad ist.

Unabhängig davon ist auch hier die Trennung nach ortho-Substitutionsgrad wirksam.

Somit eröffnet das erfindungsgemäße Verfahren auch für diese Substanzklasse neue Wege, die Herstellungsform der Rohstoffe (Aminstufe) und die Verwendungsform der Endprodukte (Isocyanatstufe) durch Fraktionierung und/oder Anreicherung auf der Aminstufe und separate Weiterverarbeitung der Fraktionen zu entkoppeln, so daß eine getrennte Optimierung beider Stufen erleichtert wird bis bin zur Gewinnung völlig neuer Isocyanatgemische oder erst möglich wird wo bislang geeignete Verfahren und Methoden fehlten oder wenig praktikabel sind.

Ergänzt werden diese "Leistungen" durch ein weiteres Selektivitätskriterium, welches bei der Fraktionierung technischer Polyamingemische, insbesondere solcher mit höherkernigen Homologen, gefunden wurde und die "Kernigkeit" der Polyamingemische betrifft.

Mit dem Begriff "Kernigkeit" wird primär die Anzahl der Aryleinheiten einer Komponente eines aromatischen Polyamingemisches ausgedrückt. Im weiteren Sinne wird der Begriff der Kernigkeit dafür verwendet, um für ein aus zahlreichen Komponenten, mit einer individuellen exakten Kernigkeit, bestehendes Polyamingemisch statistisch eine Kernigkeit des Gesamtgemisches auszudrücken.

Besonders überraschenderweise wurde nun bei der Fraktionierung von Polyamingemischen mit höherkernigen Anteilen, insbesondere bei der Fraktionierung technischer Gemische von Anilin-Formaldehydkondensaten, gefunden, daß sich die höherkernigen Komponenten in der die Fraktionierungsstufe verlassenden organischen Phase gezielt sowohl relativ anreichern als auch relativ abreichern lassen, in Abhängigkeit von der Molarität der wäßrigen Phase in der Extraktionsstufe (6).

Eine hohe Molarität der wäßrigen Phase in (6) innerhalb des angegebenen Molaritätsbereichs führt zu einer relativen Abreicherung höherkerniger Komponenten in der organischen Phase (D) und dementsprechend zu einer relativen Anreicherung in der wäßrigen Phase (H).

Eine niedrige Molarität der wäßrigen Phase (C) innerhalb des angegebenen Molaritätsbereichs führt zu einer relativen Anreicherung höherkerniger Komponenten in der organischen Phase (D).

Der überraschende Befund kann dahingehend erweitert und präzisiert werden, das die relative An- und Abreicherung auch innerhalb der höherkernigen Homologen untereinander stattfindet. Werden beispielsweise in einem technischen Gemisch des Diamino-diphenylmethans in der einen Fraktion die Dreikernkomponenten gegenüber den Zweikernkomponenten relativ an- oder abgereichert, wird auch eine relative An- oder Abreicherung von Vierkernkomponenten gegenüber Dreikernkomponenten, d.h. eine noch stärkere relative An- oder Abreicherung, gefunden, desgleichen von Fünfkernkomponenten gegenüber Vierkernkomponenten u.s.w.

Daraus und aus der gleichzeitig und stets im Sinne einer relativen Verstärkung des "ortho-Substitutionsgrades" in der organischen Phase (D) ablaufenden Isomerentrennung und aus der Möglichkeit, mit einzelnen Produktfraktionen die erfindungsgemäße Trennung, gegebenenfalls mit geänderten Verfahrensparametern, zu wiederholen, ergeben sich zahlreiche Möglichkeiten, ausgehend von bekannten und gut zugänglichen Polyamingemischen über das erfindungsgemäße Verfahren zu weniger gut zugänglichen oder völlig neuen, weil nach dem Stand der Technik bislang unzugänglichen, Polyaminen und damit Polyisocyanaten zu gelangen. Das gilt besonders für Produkte, der Diamino- und Diisocyanato-diphenylmethanreihe und ganz besonders für Polyamin - und Polyisocyanatgemische mit einem extrem hohen Anteil an höherkernigen Komponenten.

Die An- bzw. Abreicherung wird in der Regel effektiver mit steigendem Protonierungsgrad in der wäßrigen Phase der Trennstufe.

Darüber hinaus erweist sich das erfindungsgemäße Verfahren als von allgemeiner Wirksamkeit auch auf andere strukturähnliche Polyamine.

So können beispielsweise in den bereits erwähnten Polyamingemischen, die durch Nitrierung von Di- und Polyarylmethanen und anschließender Reduktion gewonnen werden, auch Monoaminopolyarylmethanverbindungen oder Komponenten enthalten sein, bei denen eine oder mehrere Methylengruppen durch Nebenreaktionen in Keto- und/oder Hydroxymethylengruppen und damit in unerwünschte Nebenprodukte umgewandelt worden sind.

Bei der Kondensation von Arylaminen mit Carbonylverbindungen können zahlreiche unvollständig umgelagerte Zwischenverbindungen und Nebenprodukte auftreten.

Die meisten dieser Verbindungen unterliegen in der Regel bei der Fraktionierung der sie enthaltenden Polyamingemische einer Anreicherung in einer der resultierenden Fraktionen, so daß der Effekt zur Abtrennung und Fraktionierung genutzt werden kann.

Gegebenenfalls können derartige Produkte auf diesem Wege angereichert werden oder als gezielt hergestellte Polyamingemische, wie z.B. Polyaminobenzophenone oder Aminobenzylarylamingemische ihrerseits fraktioniert werden.

Die die Extraktionsstufe (6) verlassende, organische Phase (D) enthält unter anderem noch geringe Mengen an Säure, im allgemeinen und in Abhängigkeit von den Verfahrensparametern in der Extraktionsstufe (6) zwischen 0,01 und 0,5 Gew.-%, die vorteilhaft vor der destillativen Aufarbeitung des Mengenstromes (D) entfernt werden.

Im einfachsten Falle geschieht dies durch Neutralisation mit überschüssigen, verdünnten wäßrigen Basen, beispielsweise verdünnter Natronlauge.

Die organische Phase (D) bzw. (D') wird, gegebenenfalls nach Durchlaufen der Waschstufe (10), in die Destillationsstufe (11) überführt.

In der letzten Stufe der gegebenenfalls mehrstufigen Destillationsstufe (11) wird das erste Polyaminteilprodukt (G) abgetrennt und im Verfahrensprodukttank (13) gesammelt.

Das entsprechende zweite Teilprodukt befindet sich in der die Extraktionsstufe (6) verlassenden wäßrigen Phase (H).

Die wäßrige Phase (H) wird gegebenenfalls nach Zusatz von Hilfsamin und/oder hydrophobem Lösungsmittel, in der Neutralisationsstufe (8) mit der wäßrigen Lösung einer starken Base, vorzugsweise konz. Natronlauge, zur Neutralisation der enthaltenen Säure umgesetzt.

Die bei der Neutralisation gebildete wäßrige Phase wird abgetrennt und im Abwasserbehälter (15) gesammelt.

Die bei der Neutralisation gebildete organische Phase wird als Mengenstrom (J) abgetrennt, gegebenenfalls in der Waschstufe (9) mit Wasser gewaschen, und der destillativen Aufarbeitung (12) zugeführt.

In der letzten Stufe der gegebenenfalls mehrstufigen Destillationsstufe (12) wird das zweite Polyaminteilprodukt (L) abgetrennt und im Verfahrensprodukttank (14) gesammelt.

Mit dieser ersten Variante des erfindungsgemäßen Verfahrens lassen sich beträchtliche Trennleistungen bei der Fraktionierung von Polyamingemischen erzielen und zahlreiche Trennprobleme zufriedenstellend lösen.

Insbesondere in der ersten Polyaminfraktion (G) kann die relative Anreicherung der in dieser Fraktion bevorzugt enthaltenen Komponenten gezielt variiert und maximiert werden.

Der in der zweiten Polyaminfraktion (L) verbleibende Anteil dieser Komponenten kann jedoch gemäß dieser ersten Variante nicht in gleicher Weise minimiert werden, sondern variabel nur bis zu einem Gehalt relativ abgereichert werden, dessen untere Grenze abhängt von dem für die jeweiligen Verfahrensparameter charakteristischen Verteilungsgleichgewicht der Polyaminkomponenten von (A) zwischen der organischen Phase (B) beim Eintritt in den Extraktor (6) und der wäßrigen Phase (H) beim Verlassen des Extraktors (6).

Durch Zumischen von Anteilen der zweiten Polyaminfraktion (L), vorzugsweise als Teilstrom (J'') von (J) zum Ausgangsarylamin (A) kann dessen Gehalt an Komponenten von (G) relativ gesenkt und damit über das Verteilungsgleichgewicht die gemäß erster Variante erreichbare Untergrenze in diesen Komponenten im zweiten Teilprodukt (L) verschoben werden. Die damit erzielte nur graduelle Verbesserung der Trennleistung mag für bestimmte Anwendungen ausreichend sein, geht in der Regel aber zu Lasten des Durchsatzes an (A).

Vorteilhafter und als Ausführungsform bevorzugt ist eine zweite Variante des erfindungsgemäßen Verfahrens, bei der sich zusätzlich auch in der zweiten Polyaminfraktion (L) die relative Anreicherung der in dieser Fraktion bevorzugt enthaltenen Komponenten weitgehend unabhängig von der ersten Produktfraktion, gezielt variieren läßt, indem die in der Extraktionsstufe (6) anfallende wäßrige Phase (H) oder zumindest eine Teilmenge derselben in einer nachgeschalteten, nach dem Gegenstromprinzip arbeitenden Extraktionsstufe (7) mit einer organischen Phase (O) extrahiert wird.

Die organische Phase (O) besteht im allgemeinen neben hydrophobem Lösungsmittel aus Hilfsamin und/oder Polyamin, letzteres vorzugsweise mit der Zusammensetzung des zweiten Verfahrensteilproduktes (L).

Bei Verwendung einer organischen Phase (O) ohne Polyamin resultiert in der die Extraktionsstufe (7) verlassenden wäßrigen Phase (N) eine Polyaminfraktion, in welcher die relative Anreicherung der in dieser Phase bevorzugt enthaltenen Komponenten über die in der wäßrigen Phase (H) erzielte Anreicherung hinaus gezielt erhöht und maximiert werden kann, auf Kosten der Polyaminkonzentration in der wäßrige Phase.

Polyamin als Bestandteil der organischen Phase (O) bewirkt, daß die die Verfahrensstufe (7) verlassenden Phasen (M) und (N) eine höhere und damit für die Durchführung des erfindungsgemäßen Verfahrens energetisch vorteilhaftere Polyaminkonzentration aufweisen, als bei Verwendung einer organischen Phase (O) ohne Polyamin.

Durch die bevorzugte Verwendung eines Polyamins mit der Zusammensetzung des zweiten Teilproduktes (L) als Bestandteil der organischen Phase (O) kann auch auf einem höheren und damit vorteilhaften Konzentrationsniveau infolge Gleichgewichtseinstellung mit Selbstverstärkung des Trenneffektes die relative Anreicherung der in der die Trennstufe (7) verlassenden wäßrigen Phase (N) bevorzugt enthaltenen Polyaminkomponenten und damit der zweiten Polyaminfraktion (L) variiert und maximiert werden.

Im einfachsten und allgemeinen Fall wird die organische Phase (O) gebildet aus zumindest einem Teilstrom von Mengenstrom (B) und gegebenenfalls weiterem Hilfsamin und/oder Polyamin, beispielsweise einer Teilmenge von (L). Vorzugsweise wird (O) gebildet aus einer Teilmenge von (B) und einer Teilmenge (J'') des Mengenstromes (J), enthaltend das Polyamingemisch (L) neben gegebenenfalls Hilfsamin und/oder gegebenenfalls hydrophobem Lösungsmittel. Durch die an anderer Stelle beschriebene mögliche Zugabe von hydrophobem Lösungsmittel und/oder Hilfsamin vor oder in der Neutralisationsstufe (8) kann eine organische Phase (J) resultieren, die in der Zusammensetzung soweit der organischen Phase (O) entspricht, daß sie, vorzugsweise nach Durchlaufen der Waschstufe (9), als Teilstrom (J'') direkt und praktisch ohne weitere Zusätze als organische Phase (O) in Verfahrensstufe (7) eingesetzt wird.

Die Molarität der in der nachgeschalteten Extraktionsstufe (7) eingesetzten wäßrigen Phase liegt im allgemeinen auf gleicher Höhe wie in der die Extraktionsstufe (6) verlassenden wäßrigen Phase (H). Grundsätzlich ist es jedoch möglich durch Zugabe von wäßriger Säure und/oder Wasser gegebenenfalls auch von Hilfsamin zur wäßrigen Phase (H) sowohl die Molarität als auch den Protonierungsgrad, zur Verbesserung der verfahrensgemäßen Trennleistung, zu verändern.

Grundsätzlich ist es auch möglich, die Molarität durch destillativen Entzug von Wasser aus (H) zu erhöhen.

Die in Stufe (7) resultierende organische Phase (M) wird der in Extraktionsstufe (6) eingesetzten organischen Phase (B) zugesetzt.

Die in Stufe (7) resultierende wäßrige Phase (N) wird der Neutralisationsstufe (8) zugeführt.

Mit der zweiten Variante des erfindungsgemäßen Verfahrens lassen sich die relative Anreicherung in beiden resultierenden Polyaminfraktionen gezielt variieren und maximieren. Neben dieser in qualitativer Hinsicht großen Vielseitigkeit und Leistungsfähigkeit bietet die zweite Verfahrensvariante zumindest für die zweite Polyaminfraktion (L) auch eine energetisch günstige Ausführungsform. Der mit Gewinnung der ersten Polyaminfraktion (G) verbundene Aufwand steigt dagegen relativ um so stärker, je geringer der mengenmäßige Anteil von (G), bezogen auf eingesetztes Polyamingemisch (A) ist, weil der verbleibende Gehalt an Polyamin (G) in der destillativ aufzuarbeitenden organischen Phase (D) entsprechend immer Kleiner wird.

Der Effekt kommt besonders dann zum Tragen, wenn die mit (G) abgetrennten Komponenten im Ausgangsgemisch (A) nur in geringer Konzentration enthalten sind und/oder relativ hoch in der Fraktion (G) angereichert werden, z.B. bei der erfindungsgemäßen Auftrennung von Polyamingemischen der Diphenylmethanreihe.

Eine teilweise Einbringung von Ausgaugspolyamin (A) in die Trennstufe (6) über die wäßrige Phase (C) bringt in der Regel eine Erhöhung der Polyaminkonzentration in (D) und damit energetische Entlastung. Bei der erfindungsgemäßen Durchführung des Verfahrens ist diese Entlastung aber infolge der Gleichgewichtseinstellung zwischen dem Polyamin in der wäßrigen Phase (C) und dem Polyamin in der organischen Phase (D) mit einer Verschlechterung des qualitativen Trennergebnisses im ersten Teilprodukt (G) verbunden, so daß von dieser Möglichkeit nur in untergeordnetem Maße oder in Fällen mit entsprechend geringen Anforderungen an das Trennergebnis Gebrauch gemacht wird.

Eine in dieser Hinsicht verbesserte Ausführung stellt die dritte Variante des erfindungsgemäßen Verfahrens dar. Ausgehend von der ersten Variante wird diese dahingehend erweitert, daß die die Verfahrensstufe (6) verlassende, das erste Teilprodukt (G) in gegenüber der Konzentration von (A) in (B) verringerter Konzentration enthaltende organische Phase (D) geteilt wird in einen Mengenstrom (D'), der weiterhin mit dem Ziel der Gewinnung der Polyaminfraktion (G) den Aufarbeitungsstufen (10) und (11) zugeführt wird, und in einen Mengenstrom (D'').

Der Mengenstrom (D'') wird in einer vorgelagerten Extraktionsstufe (5) mit zumindest einer Teilmenge, vorzugsweise mit der Gesamtmenge der als Mengenstrom (X) zur Verfügung stehenden wäßrigen Säure umgesetzt; gegebenenfalls erfolgt die Umsetzung als mehrstufige Gegenstromextraktion.

Der dem Extraktor (5) zugeführte Mengenstrom (D'') wird dabei so bemessen, daß bei der Umsetzung mit Mengenstrom (X) ein möglichst weitgehender, vorzugsweise praktisch quantitativer Übergang der in der organischen Phase (D'') enthaltenen Polyamine in die den Extraktor (5) verlassende wäßrige Phase erfolgt.

Übersteigt die Summe der in die Verfahrensstufe (5) eingebrachten Säureäquivalente die der Aminäquivalente, erfolgt der Übergang der Amine in die wäßrige Phase bereits in einer einzigen Verfahrensstufe praktisch quantitativ. Das Vorhandensein von freier Säure in der resultierenden wäßrigen Phase ist für den Fortgang des Verfahrens ohne Belang.

Auch im Falle eines Überschusses der Aminäquivalente in (D'') über die Säureäquivälente in (X), und sogar bei einem begrenzten Überschuss der Polyaminäquivalente in (D'') über die Säureäquivalente in (X), kann eine im Sinne des erfindungsgemäßen Verfahrens ausreichend an Amin und insbesondere an Polyamin verarmte organische Phase (P) gewonnen werden durch Mehrstufigkeit der vorgelagerten Verfahrensstufe (5) und Arbeiten im Gegenstrom.

Im übrigen richtet sich der in (P) zulässige Höchstgehalt an Amin und insbesondere der Gehalt an Polyamin nach den aus der jeweiligen Trennaufgabe resultierenden qualitativen Anforderungen an die Verfahrensprodukte, d.h. die Qualität der Trennung, im Falle der Variante 3 insbesondere an das Verfahrensteilprodukt (L). Die Einhaltung des für die Qualität von (L) relevanten Gehaltes an Polyamin wird im Rahmen der technischen Gegebenheiten unter Ausschöpfung des zur Verfügung stehenden Säurepotentials über die Bemessung des Teilmengenstroms (D'') kontrolliert.

Der Restgehalt an Polyamin in der die Verfahrensstufe (5) verlassenden organischen Phase (P) liegt im allgemeinen bei <5 Gew.-%, vorzugsweise bei <1 Gew.-%.

In quantitativer Hinsicht kommt es dem Verfahren und insbesondere der vorgelagerten Verfahrensstufe (5) zustatten, daß das Verhältnis von (D'') zu (D') besonders dann zu höheren Werten tendiert, d.h. daß (D'') mengenmäßig größer wird, wenn das Mengenverhältnis der Polyaminfraktionen (G) zu (L) kleiner wird, weil (L) auf Kosten von (G) größer wird. Mit zunehmenden Anteil der zweiten Polyaminfraktion (L) erhöht sich die eingesetzte und damit in (5) zur Extraktion von (D'') zur Verfügung stehenden Säuremenge (X).

Die an Amin verarmte, insbesondere von Polyamin praktisch befreite, die Verfahrensstufe (5) verlassende organische Phase (P) wird der organischen Phase (B) zugeschlagen und zusammen mit dieser als Lösungsmittel für Ausgangspolyamin (A) der Verfahrensstufe (6) zugeführt.

Die die Verfahrensstufe (5) verlassende wäßrige Phase (Q) enthält neben der eingesetzten wäßrigen Säure Polyamin, welches in seiner Zusammensetzung weitgehend den in (D) abgetrennten und als Fraktion (G) isolierten Polyamin entspricht und gegebenenfalls Hilfsamin.

Im einfachsten Falle wird die die Verfahrensstufe (5) verlassende wäßrige Phase direkt als Mengenstrom (C) der Verfahrensstufe (6) zugeführt; gegebenenfalls werden wäßrige Säure und/ oder Wasser (Y) und/oder Hilfsamin zugemischt, vorzugsweise über den Mischer (6A).

Auch die Zugabe einer begrenzten Menge von Ausgangspolyamin (A) zu der wäßrigen Phase (Q) ist möglich ohne Einbuße an qualitativer Trennleistung (relativer Anreicherung) gegenüber Variante 1, dafür aber bei verbesserter quantitativer Leistung (größerer Wirtschaftlichkeit).

Bei einer weiteren vierten Variante wird die vorgelagerte Verfahrensstufe (5) und die sich ergebenden Vorteile kombiniert mit der als Variante 2 beschriebenen Ausführungsform des erfindungsgemäßen Verfahrens, die dadurch bezüglich des ersten Teilproduktes eine analoge Verbesserung erfährt wie bei Variante 3. Als eine zusätzliche Erleichterung und Vereinfachung für die Durchführung der Verfahrensstufe (5) erweist es sich, daß die Anforderung an den verbleibenden Polyamingehalt der in (5) resultierenden organischen Phase (P) Weniger streng sind, da die nachteiligen Auswirkungen eines erhöhten Polyamingehaltes in (P) auf die Qualität des zweiten Veifahrensteilproduktes (L) durch die nachgeschaltete Extraktionsstufe (7) kompensiert werden können.

Der Gehalt an Polyamin in der die Verfahrensstufe (5) verlassenden organischen Phase (P) liegt daher im allgemeinen bei <5 Gew.-%, vorzugsweise bei <3 Gew.-%.

### Beispiel 1

In einem Mischer (6A) werden 1,264 kg/h 30 %ige Salzsäure (Mengenstrom X) und 7,315 kg/h Wasser (Mengenstrom Y) mit 1,671 kg/h Anilin (Teilmenge von Mengenstrom von F und K), miteinander vermischt unter Bildung des Mengenstromes (C):
- Mengenstrom (C) ( 10,250 kg/h ): 11,0 % Anilin
3,7 % Chlorwasserstoff
80,0 % Wasser.

Mengenstrom (C) wird in einem mehrstufig wirkenden Extraktor (6) bei 90°C einer organischen Phase entgegengeführt, welche gebildet wird durch Vermischen von Ausgangspolyamin (A) mit den Mengenströmen (B) und (M) unter Zusatz von weiteren 0,362 kg/h Anilin (Teilmenge von Mengenstrom von F und K).
Mengenstrom (A)
( 2,066 kg/h )
- Mengenstrom (B) ( 3,705 kg/h ): 24,5 % Anilin
75,2 % Xylol
0,3 % Wasser.
Mengenstrom (M)
( 1,565 kg/h )

Die dabei resultierende, die Extraktionsstufe (6) verlassende, organische Phase (Mengenstrom D) hat folgende Zusammensetzung:
- Mengenstrom (D) ( 7,500 kg/h ): 20,0 % Polyarylamin
32,1 % Anilin
45,4 % Xylol
0,1 % Chlorwasserstoff
2,4 % Wasser.

Mengenstrom (D) Wird in der Neutralisationsstufe (10) mit überschüssiger verdünnter Natronlauge (Teilmengenstrom von Z und Wasser aus 2) gewaschen. Die wäßrige Phase wird als Abwasser in Tank (15) gesammelt.

Von dem gewaschenen Mengenstrom (D) wird in einer ersten Destillationsstufe (11.1) eine erste Fraktion abdestilliert (Mengenstrom E), welche praktisch das gesamte Wasser und Xylol und einen Teil des Anilins enthält.
- Mengenstrom (E) ( 4,532 kg/h ): 24,5 % Anilin
75,2 % Xylol
0,3 % Wasser.

Der größte Teil von Mengenstrom (E) wird als Mengenstrom (B) der Extraktionsstufe (6) zugeführt. Der Rest von 0,827 kg/h wird zur Bildung von Mengenstrom (O) verwendet.

Der Destillationssumpf von (11.1) wird in einer zweiten Destillationsstufe von dem verbliebenen Anilin befreit. Das Destillat (Mengenstrom F) von 1,298 kg/h wird zusammen mit dem Destillat (K) der Destillationstufe (12) wiederverwendet.

Der in Destillationsstufe (11) resultierende Rückstand besteht aus Polyamingemisch, welches als Mengenstrom (G) mit 1,500 kg/h in Tank (13) gesammelt wird.

Die den Extraktor (6) verlassende wäßrige Phase (H) hat folgende durchschnittliche Zusammensetzung
- Mengenstrom (H) ( 10,448 kg/h ): 11,2 % Polyarylamin
8,0 % Anilin
3,6 % Chlorwasserstoff
77,2 % Wasser
und wird in dem mehrstufig wirkenden Extraktor (7) bei 90°C einer organischen Phase (O) entgegengeführt, welche aus einer Teilmenge von Mengenstrom (E) und dem Mengenstrom (J''), einer Teilmenge von Mengenstrom (J) gebildet wird. Mengenstrom (O)
( 1,471 kg/h )

Die in der Extraktionsstufe (7) resultierende organische Phase (M) hat folgende durchschnittliche Zusammensetzung:
- Mengenstrom (M) ( 1,565 kg/h ): 38,3 % Polyarylamin
19,6 % Anilin
39,7 % Xylol
≤0,1 % Chlorwasserstoff
2,3 % Wasser.

Mengenstrom (M) wird der Verfahrensstufe (6) zugeführt.

Die in Verfahrensstufe (7) resultierende wäßrige Phase Mengenstrom (N) hat folgende durchschnittliche Zusammensetzung
- Mengenstrom (N) ( 10,354 kg/h ): 8,1 % Polyarylamin
10,6 % Anilin
3,6 % Chlorwasserstoff
77,7 % Wasser
und wird in der Neutralisationsstufe (8) mit überschüssiger wäßriger Natronlauge aus Tank (3) (Teilmengenstrom Z) neutralisiert. Die wäßrige, salzhaltige Phase wird abgetrennt und im Abwassertank (15) gesammelt.

Die organische Phase wird anschließend in der Waschstufe (9) mit Wasser aus Tank (2) weitgehend salzfrei gewaschen. Das Waschwasser wird ebenfalls im Abwassertank (15) gesammelt.

Die salzfrei gewaschene organische Phase (Mengenstrom J) hat folgende durchschnittliche Zusammensetzung
- Mengenstrom (J) ( 1,958 kg/h ): 43,1 % Polyarylamin
55,9 % Anilin
1,0 % Wasser
und wird in der Destillationsstufe (12) aufgetrennt in eine Destillatfraktion (Mengenstrom K) mit 0,748 kg/h, die nach mechanischer Abtrennung von Wasser mit der Destillatfraktion (F) vereinigt in Teilströmen den Stufen (6A) und (6) zugeführt wird.

Der Destillationsrückstand von (12) bildet die zweite Polyaminfraktion (L) mit 0,566 kg/h und wird im Tank (14) gesammelt.

| **Polyarylamin** | **A** [Gew. - %] | **G** [Gew. - %] | **L** [Gew. %] |
|---|---|---|---|
| **GC:** | | | |
| 2,2' Diamino-diphenylmethan | 0,90 | 1,24 | ---- |
| 2,4'-Diamino-diphenylmethan | 11,10 | 15,29 | <0,10 |
| 4,4'-Diamino-diphenylmethan | 55,57 | 38,99 | 99,50 |
| N-Methyl-4,4'-diamino-diphenylmethan | 0,43 | 0,59 | ---- |
| Σ Diamino-diphenylmethane | 68,00 | 56,11 | 99,50 |
| Σ Mehrkernpolyamine | 32,00 | 43,89 | 0,50 |
| Mengenverteilung | 100 % | 72,60 % | 27,40 % |

## Patentansprüche

1. Verfahren zur Fraktionierung von aromatischen Polyamingemischen der Diphenylmethanreihe, dadurch gekennzeichnet, daß man
a) das Polyaminausgangsgemisch (A) in einem zweiphasigen System, bestehend aus (i) einer hydrophoben Lösungsmittelphase (B), die im wesentlichen aus hydrophobem Lösungsmittel, besteht, und (ii) einer wäßrigen Phase (C), bestehend im wesentlichen aus wäßriger Lösung einer starken Säure und zumindest teilweise in der Salzform vorliegendem Hilfsamin, welches in Wasser praktisch unlöslich ist und unter Normaldruck einen mindestens 20°C unter dem Siedepunkt der am niedrigsten siedenden Komponente des Ausgangsgemisches und mindestens 20°C oberhalb des Siedepunktes des Lösungsmittels liegenden Siedepunkt aufweist, unter Zuhilfenahme einer nach dem Gegenstromprinzip arbeitenden Extraktionsstufe (6) unter Durchmischung der Phasen verteilt, indem man das Ausgangspolyamingemisch über die organische Phase (B) in die Extraktionsstufe (6) einbringt, mit der Maßgabe, daß in diesem zweiphasigen System die Summe der in den Mengenströmen (A), (B) und (C) eingebrachten Aminäquivalente die Anzahl der im Mengenstrom (C) eingebrachten Säureäquivalente stets übersteigt, und die diese Extraktionsstufe verlassende organische Phase (D) zumindest teilweise, in einer Destillationsstufe (11) in eine erste, in der Extraktionsstufe (6) wiederverwendete Fraktion (E), bestehend im wesentlichen aus hydrophobem Lösungsmittel eine zweite Fraktion (F), bestehend im wesentlichen aus Hilfsamin und eine als Destillationsrückstand (G) anfallende erste Polyaminfraktion auftrennt, und die die erste Extraktionsstufe (6) verlassende wäßrige Phase (H)
b) in eine Neutralisationsstufe (8) leitet, mit Basen, die in der wäßrigen Phase enthaltene Säure neutralisiert und anschließend in einem Phasentrennschritt in eine wäßrige Phase, enthaltend die Säure in Form ihrer neutralen Salze und eine organische Phase, enthaltend im wesentlichen Polyamin, mechanisch auftrennt und
c) die in der Neutralisationsstufe (8) anfallende organische Phase (J) zumindest teilweise in einer Destillationsstufe (12) aufarbeitet in eine Destillatfraktion (K), enthaltend die in (J) bzw. (J') enthaltenen Anteile an hydrophobem Lösungsmittel und das in (J) bzw. (J') enthaltene Hilfsamin, und in eine als Destillationsrückstand (L) anfallende zweite Polyaminfraktion.

2. Verfahren gemäß Anspruch 1, dadurch gekennzeichnet, daß man
b) die in der Extraktionsstufe (6) anfallende wäßrige Phase (H) zumindest teilweise in einer nachgeschalteten, nach dem Gegenstromprinzip arbeitenden Extraktionsstufe (7) extrahiert unter Verwendung einer organischen Phase (O) als Extraktionsmittel, bestehend neben hydrophobem Lösungsmittel aus Hilfsamin und/oder Polyamin, letzteres mit der Zusammensetzung des zweiten Teilproduktes (L) und eingebracht als Teilmenge (J") des Mengenstroms (J), die in Verfahrensstufe (7) resultierende organische Phase (M) den Mengenstrom (B) zuschlägt und damit der Extraktionsstufe (6) und die in (7) resultierende wäßrige Phase (N) der Neutralisationsstufe (8) zuführt.

3. Verfahren gemäß Anspruch 1 oder 2, dadurch gekennzeichnet, daß man die die Extraktionsstufe (6) verlassende organische Phase zumindest teilweise in einer (6) vorgelagerten Stufe (6A) mit der wäßrigen Säure (Mengenstrom X) und Wasser (Mengenstrom Y) und/oder Hilfsamin vermischt, und von dem resultierenden Gemisch nach Auftrennung in eine organische und eine wäßrige Phase wenigstens die wäßrige Phase der Verfahrensstufe (6) und die abgetrennte organische Phase dem organischen Mengenstrom (D) zuführt.

4. Verfahren gemäß einem oder mehreren der Ansprüche 1 bis 3, dadurch gekennzeichnet, daß man eine Teilmenge (D") der die Extraktionsstufe (6) verlassenden organischen Phase (D) abtrennt und in einer mehrstufigen, in der ersten Stufe vorzugsweise als Mischer-Scheidereinheit betriebenen Extraktionsstufe (5) mit dem gesamten Mengenstrom (X) der wäßrigen Säure im Gegenstrom extrahiert, und den Teilmengenstrom (D") so bemißt, daß dabei ein möglichst weitgehender Übergang des in (D") enthaltenen Polyamins in die die Extraktionseinheit (5) verlassende wäßrige Phase (Q) stattfindet, besagte wäßrige Phase (Q) anteilig oder insgesamt direkt und/oder über den Mischer (6A), aus Mengenstrom (Y) und/oder Hilfsamin und/oder weiterer wäßriger Säure als wäßrige Phase (C) der Extraktionsstufe (6) zuführt, die in (5) anfallende organische Phase (P), bestehend im wesentlichen aus hydrophobem Lösungsmittel und Hilfsamin, ebenfalls der der Extraktionsstufe (6) zugeführten der organischen Phase (B) zuschlägt und als Lösungsmittel für das Ausgangspolyamin (A) verwendet.

5. Verfahren gemäß einem oder mehreren der Ansprüche 1 bis 4, dadurch gekennzeichnet, daß man als Hilfsamin Anilin verwendet.

6. Verfahren gemäß einem oder mehreren der Ansprüche 1 bis 4, dadurch gekennzeichnet, daß man als Hilfsamin 2,6-Dimethylanilin verwendet.

7. Verfahren gemäß einem oder mehreren der Ansprüche 1 bis 4, dadurch gekennzeichnet, daß man als Hilfsamin Xylidingemische verwendet.

8. Verfahren gemäß einem oder mehreren der Ansprüche 1 bis 4, dadurch gekennzeichnet, daß man als Hilfsamin 2-Methyl-6-ethylanilin verwendet.

9. Verfahren gemäß einem oder mehreren der Ansprüche 1 bis 8, dadurch gekennzeichnet, daß man als Polyamingemisch der Diphenylmethanreihe ein Polyamingemisch verwendet, wie es bei der säurekatalysierten Anilin/Formaldehyd-Kondensation anfällt.

10. Verfahren zur Herstellung aromatischer Polyisocyanatgemische durch ein Verfahren enthaltend die folgenden Verfahrensstufen:
A) Herstellung aromatischer Polyamingemische der Diphenylmethanreihe gemäß den Ansprüchen 1-9,
B) Umwandlung der erhaltenen aromatischen Polyamingemische der Diphenylmethanreihe der Stufe (A) in die entsprechenden aromatischen Polyisocyanatgemische.

11. Verfahren zur Herstellung kernhydrierter Polyamine oder vernetzer und Epoxihärter durch ein Verfahren enthaltend die folgenden Verfahrensstufen:
A) Herstellung aromatischer Polyamingemische der Diphenylmethanreihe gemäß den Ansprüchen 1-9,
B) Umwandlung der erhaltenen aromatischen Polyamingemische der Diphenylmethanreihe der Stufe A in die entsprechenden kernhydrierter Polyamine oder Vernetzer und Expoxidhärter.

12. Verfahren zur Herstellung Polyurethankunststoffe durch ein Verfahren enthaltend die folgenden Verfahrensstufen:
A) Herstellung aromatischer Polyamingemische der Diphenylmethanreihe gemäß den Ansprüchen 1-7,
B) Umwandlung der erhaltenen aromatischen Polyamingemische der Diphenylmethanreihe der Stufe (A) in die entsprechenden Polyurethankunststoffe.

## Claims

1. A process for fractionating mixtures of aromatic polyamines from the diphenylmethane series, characterised in that
a) the initial polyamine mixture (A) is partitioned in a two-phase system consisting of (i) a hydrophobic solvent phase (B), substantially consisting of hydrophobic solvent, and (ii) an aqueous phase (C) substantially consisting of an aqueous solution of a strong acid and an auxiliary amine which is at least partly in the salt form, which is virtually insoluble in water and has a boiling point at atmospheric pressure at least 20°C below the boiling point of the component with the lowest boiling point in the initial mixture and at least 20°C above the boiling point of the solvent, using an extraction stage (6) operating on the counterflow principle with thorough mixing of the phases, by introducing the initial polyamine mixture to extraction stage (6) via organic phase (B), with the proviso that the sum of the amine equivalents introduced to this two-phase system in streams (A), (B) and (C) always exceeds the number of acid equivalents introduced in stream (C), and the organic phase (D) leaving this extraction stage is at least partly separated in a distillation stage (11), into a first fraction (E) substantially consisting of hydrophobic solvent, which is recycled to extraction stage (6), a second fraction (F) substantially consisting of auxiliary amine and a first polyamine fraction produced as distillation residue (G), and the aqueous phase (H) leaving the first extraction stage (6)
b) passes to a neutralisation stage (8), the acid contained in the aqueous phase being neutralised with bases, and is then mechanically separated in a phase separation step into an aqueous phase containing the acid in the form of its neutral salts and an organic phase substantially containing polyamine and
c) the organic phase (J) produced in neutralisation stage (8) is at least partly worked up in distillation stage (12) into a distillate fraction (K) containing the amount of hydrophobic solvent contained in (J) or (J') and the auxiliary amine contained in (J) or (J') and a second polyamine fraction produced as distillation residue (L).

2. A process according to Claim 1, characterised in that
b) the aqueous phase (H) produced in extraction stage (6) is at least partly extracted in a downstream extraction stage (7) operated on the counterflow principle, using an organic phase (O) as extraction agent, consisting, in addition to hydrophobic solvent, of auxiliary amine and/or polyamine, the latter having the composition of the second subproduct (L) and being introduced as one constituent (J") of stream (J), the organic phase (M) resulting from process stage (7) being added to stream (B) and thus taken to extraction stage (6) and the aqueous phase (N) resulting from (7) being taken to neutralisation stage (8).

3. A process according to Claim 1 or 2, characterised in that at least some of the organic phase leaving extraction stage (6) is mixed, in a stage (6A) which is upstream of (6), with the aqueous acid (stream X) and water (stream Y) and/or auxiliary amine and, after separating into an organic and an aqueous phase,at least the aqueous phase from the resulting mixture is taken to process stage (6) and the separated organic phase is taken to organic stream (D).

4. A process according to one or more of Claims 1 to 3, characterised in that a substream (D") is separated from organic phase (D) leaving extraction stage (6) and is extracted in a multi-stage extraction stage (5), whose first stage is preferably operated as a mixer-settler unit, in counterflow with the entire amount of stream (X) consisting of aqueous acid and the size of substream (D") is selected so that as extensive a transfer as possible of polyamine contained in (D") to aqueous phase (Q) leaving extraction stage (5) takes place, part of or all of said aqueous phase (Q) being taken to extraction stage (6) directly and/or via mixer (6A), from stream (Y) and/or auxiliary amine and/or further aqueous acid, as aqueous phase (C), the organic phase (P) produced in (5), substantially consisting of hydrophobic solvent and auxiliary amine, likewise being added to organic phase (B) being supplied to extraction stage (6) and used as solvent for the initial polyamine (A).

5. A process according to one or more of Claims 1 to 4, characterised in that aniline is used as auxiliary amine.

6. A process according to one or more of Claims 1 to 4, characterised in that 2,6-dimethylaniline is used as auxiliary amine.

7. A process according to one or more of Claims 1 to 4, characterised in that xylidine mixtures are used as auxiliary amine.

8. A process according to one or more of Claims 1 to 4, characterised in that 2-methyl-6-ethylaniline is used as auxiliary amine.

9. A process according to one or more of Claims 1 to 8, characterised in that a polyamine mixture like the one produced during acid-catalysed aniline/formaldehyde condensation is used as a polyamine mixture from the diphenylmethane series.

10. A process for preparing mixtures of aromatic polyisocyanates by a process which includes the following process steps:
A) preparation of a mixture of aromatic polyamines from the diphenylmethane series in accordance with Claims 1 - 9,
B) conversion of the mixture of aromatic polyamines from the diphenylmethane series obtained in step (A) into the corresponding mixture of aromatic polyisocyanates.

11. A process for preparing ring-hydrogenated polyamines or cross-linking agents and epoxide hardeners by a process which includes the following process steps:
A) preparation of a mixture of aromatic polyamines from the diphenylmethane series in accordance with Claims 1 - 9,
B) conversion of the mixture of aromatic polyamines from the diphenylmethane series obtained in step (A) into the corresponding ring-hydrogenated polyamines or cross-linking agents and epoxide hardeners.

12. A process for preparing polyurethane plastics by a process which includes the following process steps:
A) preparation of a mixture of aromatic polyamines from the diphenylmethane series in accordance with Claims 1 - 7,
B) conversion of the mixture of aromatic polyamines from the diphenylmethane series obtained in step (A) into the corresponding polyurethane plastics.

## Revendications

1. Procédé de fractionnement et de purification de mélanges de polyamines aromatiques de la série du diphénylméthane, caractérisé en ce que
a) on sépare le mélange de départ de polyamines (A) dans un système à deux phases constitué
(i) d'une phase de solvant hydrophobe (B), qui est constituée principalement d'un solvant hydrophobe et éventuellement d'une amine auxiliaire aromatique, qui est pratiquement insoluble dans l'eau et qui présente sous pression normale, un point d'ébullition se trouvant au moins 20 °C sous le point d'ébullition des composants du mélange de départ ayant le point d'ébullition le plus bas et d'au moins 20 °C au-dessus du point d'ébullition du solvant, et/ou éventuellement de polyamines, et
(ii) d'une phase aqueuse (C) constituée principalement d'eau, d'un acide fort et éventuellement d'une amine auxiliaire se trouvant au moins en partie sous forme de sel, ainsi qu'éventuellement des polyamines, se trouvant au moins en partie sous forme de sel, au moyen d'une étape d'extraction (6) travaillant selon le principe à contre-courant par mélange des phases, en ce qu'on amène le mélange de polyamines de départ de préférence au-dessus de la phase organique (B) dans l'étape d'extraction (6) avec la condition que dans ce système à deux phases la somme des équivalents amine amenés dans les débits de courants (A), (B) et (C) est toujours supérieure au nombre d'équivalents acide, amenés dans le débit de courant (C), qui sépare cette phase organique (D) quittant l'étape d'extraction, au moins en partie, éventuellement après réalisation d'une étape de lavage (10), dans une étape de distillation (11) effectuée de préférence en plusieurs étapes, dans le cas de l'utilisation simultanée préférée de l'amine auxiliaire, en une première fraction recyclée (E) dans l'étape d'extraction (6), constituée principalement du solvant hydrophobe et éventuellement de l'amine auxiliaire, éventuellement une deuxième fraction (F), constituée principalement de l'amine auxiliaire et éventuellement du solvant hydrophobe et une première fraction de polyamine présente comme résidu de distillation (G), et la phase aqueuse (H) quittant la première étape d'extraction (6)
b) on amène, dans une étape de neutralisation (8), avec des bases, de préférence de la lessive de soude aqueuse, qui neutralise les acides contenus dans la phase aqueuse et finalement dans une étape de séparation de phases, on sépare mécaniquement une phase aqueuse, contenant les acides sous forme de leurs sels neutres et une phase organique contenant principalement la polyamine, éventuellement l'amine auxiliaire et éventuellement de faibles quantités de solvant hydrophobe et
c) on finit le traitement de la phase organique (J) se formant dans l'étape de neutralisation (8) éventuellement après avoir effectué une étape de lavage (9), au moins en partie, dans une étape de distillation (12), en une fraction de distillation (K), contenant la partie de solvant hydrophobes contenue dans (J) respectivement (J') et l'amine auxiliaire contenue éventuellement dans (J) respectivement (J'), et en une deuxième fraction de polyamine se formant comme résidu (L) de distillation.

2. Procédé selon la revendication 1, caractérisé en ce que
b) on extrait la phase aqueuse (H) se formant dans l'étape d'extraction (6) au moins en partie dans une étape d'extraction (7) placée en aval, travaillant selon le principe à contre-courant, en utilisant une phase organique (O) comme moyen d'extraction, constituée en plus du solvant hydrophobe de l'amine auxiliaire et/ou de la polyamine, de préférence cette dernière étant ajoutée avec la composition du deuxième produit partiel (L) et de préférence amené comme quantité partielle (J") du débit de courant (J), on ajoute au débit de courant (B) la phase organique (M) résultant de l'étape de procédé (7) et ainsi, on l'amène à l'étape d'extraction (6) et on amène à l'étape de neutralisation (8) la phase aqueuse (N) résultante de (7).

3. Procédé selon la revendication 1 ou 2, caractérisé en ce qu'on mélange la phase organique quittant l'étape d'extraction (6) au moins en partie dans une étape (6A) précédant (6) avec l'acide aqueux (débit de courant X) et éventuellement l'eau (débit de courant Y) et/ou éventuellement l'amine auxiliaire et qu'on amène le mélange résultant, après séparation en une phase organique et une phase aqueuse, au moins la phase aqueuse de l'étape du procédé (6) et la phase organique éventuellement séparée du débit de courant (D) organique.

4. Procédé selon une ou plusieurs des revendications 1 à 3, caractérisé en ce qu'on sépare une quantité partielle (D") de la phase organique (D) quittant l'étape d'extraction (6) et on extrait à contre-courant dans une étape d'extraction (5) éventuellement à plusieurs étapes, fonctionnant dans la première étape de préférence comme une unité de mélange-séparation, avec au moins une quantité partielle, de préférence, avec le débit de courant total (X) de l'acide aqueux, éventuellement avec addition de l'amine auxiliaire, et on mesure le débit de courant partiel (D") de manière qu'un transfert ait ainsi lieu, si possible poussé, de la polyamine contenue dans (D") dans la phase aqueuse (Q) quittant l'unité d'extraction (5), ladite phase aqueuse (Q) étant amenée partiellement ou totalement, directement et/ou sur le mélangeur (6A), éventuellement après addition d'eau provenant du débit de courant (Y) et/ou l'amine auxiliaire et/ou un autre acide aqueux comme phase aqueuse (C) de l'étape d'extraction (6), qui est ajouté à la phase organique (P) se formant en (5), constituée principalement du solvant hydrophobe et éventuellement de l'amine auxiliaire, de la même manière, on utilise la phase organique (B) amenée à l'étape d'extraction (6) et utilisée comme solvant pour la polyamine (A) de départ.

5. Procédé selon une ou plusieurs des revendications 1 à 4, caractérisé en ce qu'on utilise l'aniline comme amine auxiliaire.

6. Procédé selon une ou plusieurs des revendications 1 à 4, caractérisé en ce qu'on utilise la 2,6-diméthylaniline comme amine auxiliaire.

7. Procédé selon une ou plusieurs des revendications 1 à 4, caractérisé en ce qu'on utilise un mélange de xylidine comme amine auxiliaire.

8. Procédé selon une ou plusieurs des revendications 1 à 4, caractérisé en ce qu'on utilise la 2-méthyl-6-éthylaniline comme amine auxiliaire.

9. Procédé selon une ou plusieurs des revendications 1 à 8, caractérisé en ce qu'on utilise comme mélange de polyamines un mélange de polyamines de la série du diphénylméthane qui est obtenu par la condensation catalytique acide d'aniline/formaldéhyde.

10. Procédé de préparation de mélanges de polyisocyanates aromatiques par un procédé comportant les étapes de procédé suivantes :
A) la préparation de mélanges de polyamines aromatiques de la série du diphénylméthane selon les revendications 1 à 9,
B) la transformation des mélanges de polyamines aromatiques de la série du diphénylméthane obtenus dans l'étape (A) en mélanges de polyisocyanates aromatiques correspondants.

11. Procédé de préparation de polyamines à noyaux hydrogénés ou de réticulants et de durcisseurs époxy par un procédé comprenant les étapes de procédé suivantes :
A) la préparation de mélanges de polyamines aromatiques de la série du diphénylméthane selon les revendications 1 à 9,
B) la transformation des mélanges de polyamines aromatiques de la série du diphénylméthane obtenus dans l'étape (A) en polyamine à noyaux hydrogénés ou réticulants et durcisseurs époxydes correspondants.

12. Procédé de préparation de matière synthétique de polyuréthane par un procédé contenant les étapes de procédé suivantes :
A) la préparation de mélanges de polyamines aromatiques de la série du diphénylméthane selon les revendications 1 à 9,
B) la transformation des mélanges de polyamines aromatiques de la série du diphénylméthane obtenus dans l'étape (A) en matière synthétique de polyuréthane correspondante.
